# EUROPÄISCHE PATENTSCHRIFT

(11) **EP 1 778 166 B1**
(45) Veröffentlichungstag und Bekanntmachung des Hinweises auf die Patenterteilung: **28.04.2010**
(21) Anmeldenummer: 05752271.6
(22) Anmeldetag: 16.06.2005
(51) Int. Cl.: A61Q 5/08, A61K 8/25, A61K 8/38

(54) **STAUBARME BLONDIERPULVER**
LOW-DUST BLONDING POWDER
POUDRE DECOLORANTE PRODUISANT PEU DE POUSSIERE

(30) Priorität: 20.08.2004 DE 102004040332
(43) Veröffentlichungstag der Anmeldung: 02.05.2007
(73) Patentinhaber: Henkel AG & Co. KGaA, 40589 Düsseldorf (DE)
(72) Erfinder: HÖFFKES, Horst, 40595 Düsseldorf (DE); SEILER, Martina, 47228 Duisburg (DE)
(86) Internationale Anmeldenummer: PCT/EP2005/006457
(87) Internationale Veröffentlichungsnummer: WO 2006/021252

(56) Entgegenhaltungen:
- EP-A- 0 560 088
- EP-A- 0 574 696
- EP-A- 0 583 767
- EP-A- 1 493 427
- WO-A-2004/104154
- DE-A1- 2 013 763
- DE-A1- 3 814 356
- HASENZAHL S,, BRAUNAGEL A.: SÖFW-JOURNAL, Bd. 129, Nr. 8, 2003, Seiten 1-8, XP002340152

## Beschreibung

Die vorliegende Erfindung betrifft pulverförmige Zusammensetzungen, welche neben mindestens einer Peroxoverbindung zusätzlich partikelförmiges Siliziumdioxid, welches Poren aufweist, mit einem mittleren Teilchendurchmesser von mindestens 20 µm und einer BET-Oberfläche von 40 bis 400 m²/g (bestimmt nach DIN 66131 mit Stickstoff) enthalten und deren Herstellverfahren, die Verwendung des ausgewählten partikelförmigen Siliziumdioxids zur Entstaubung von Blondierpulvern, ein Verfahren zur Herstellung eines anwendungsbereiten Bleichmittels für keratinhaltige Fasern, sowie ein Mittel zur Aufhellung keratinhaltiger Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus.

Ferner werden häufig aufhellende Verfahren, die sogenannten Blondierverfahren, angewendet. Die in den Bleichmitteln enthaltenen Oxidationsmittel wirken auf den natürlichen Haarfarbstoff Melanin und gegebenenfalls auf in der Faser befindliche synthetische Farbstoffe oxidativ ein und verursachen dadurch eine Aufhellung der Haarfarbe. Die Grundlagen der Blondier- und oxidativen Färbeverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Aufhellen bzw. Blondieren menschlicher Haare - insbesondere für die Strähnchenapplikation - werden üblicherweise feste oder pastenförmige Zubereitungen mit festen Oxidationsmitteln unmittelbar vor der Anwendung mit einer Wasserstoffperoxid-haltigen Zusammensetzung vermischt. Letztere Zusammensetzung liegt häufig als Lotion, O/W- oder W/O-Emulsion vor.
Die resultierende Anwendungsmischung, im weiteren als "Bleichmittel" bezeichnet, wird dann auf das Haar aufgebracht und nach einer bestimmten Einwirkzeit wieder ausgespült.

Wenn in dem zuvor beschriebenen Mischvorgang feste Zubereitungen verwendet werden, so liegen diese meist als Pulver vor. Pulverförmige Feststoffe bergen den Nachteil, dass sie bei der Anwendung stauben und somit der Staub durch den Anwender eingeatmet werden kann. Des weiteren besteht der Nachteil, dass die Pulver während des Auflösevorgangs in der Wasserstoffperoxid-haltigen Zusammensetzung verklumpen. Je nach Beschaffenheit des Pulvers kann bei Zumischung zu der Wasserstoffperoxid-haltigen Zusammensetzung eine plötzliche Zersetzungsreaktion des Wasserstoffperoxids unter starker Gasbildung einsetzen.

Die Entstaubung wird bekanntermaßen bei der Herstellung der Blondierpulver in einem zusätzlichen Verfahrensschritt gewährleistet. Beispielsweise kann die Staubbildung durch Besprühen des Pulvers beispielsweise mit flüssigen Ölen oder Wachsen gehemmt werden. Diesen Vorgang kennt der Fachmann u.a. aus der Druckschrift EP-B1-560 088.

Ferner ist es möglich, das Pulver mit einem polymeren Bindemittel zu agglomerieren und auf diese Art und Weise die Staubbildung zu verringern. Diese Entstaubungsmethode wird beispielsweise in der Druckschrift EP-A2-574 696 offenbart.

Die Offenlegungsschrift WO-A1-03/037287 betrifft Kosmetika, welche mittels Pyrolyse hergestelltes Siliziumdioxid mit einem mittleren Teilchendurchmesser von 10 bis 120 µm und einer BET-Oberfläche von 40 bis 400 m²/g (bestimmt nach DIN 66131 mit Stickstoff) enthalten.

In der EP-A1-727 037 werden Siliziumdioxid-Partikel mit einer definierten Porengröße offenbart.

DE-3814356-A1 beschreibt Mittel zum Bleichen von Haaren, die Kaliumpersulfat, Ammoniumpersulfat und disperse Kieselsäure (Aerosil 200 ®) enthalten.

Aufgabe der vorliegenden Erfindung ist es, die pulverförmigen Blondiermittel staubfrei zu gestalten und zu diesem Zweck ein kostengünstiges Verfahren bereitzustellen.

Es wurde nun überraschenderweise gefunden, daß in einem erfindungsgemäßen Entstaubungsverfahren unter Verwendung der erfindungsgemäß verwendeten Siliziumdioxid-Partikel Blondierpulver staubfrei herstellbar sind, ohne einen kostenintensiven Zusatzschritt wie, z.B. Besprühen des Pulvers mit einem Öl oder Beschichtung des Pulvers mit einem Polymer, zurückgreifen zu müssen.

Ein erster Gegenstand der vorliegenden Erfindung ist daher ein Verfahren zur Entstaubung von Blondierpulvern, in dem ein Pulver, enthaltend mindestens eine Peroxoverbindung, mit einem partikelförmigen Siliziumdioxid vermengt wird, wobei das partikelförmige Siliziumdioxid Poren aufweist und einen mittleren Teilchendurchmesser von mindestens 20 µm und eine BET-Oberfläche von 40 bis 400 m²/g (bestimmt nach DIN 66131 mit Stickstoff) besitzt.

Bei dem partikelförmigen Siliziumdioxid handelt es sich bevorzugt um pyrogenes Siliziumdioxid.

Es ist besonders bevorzugt, wenn das partikelförmige Siliziumdioxid einen mittleren Teilchendurchmesser von mindestens 40 µm, ganz besonders bevorzugt von mindestens 100 µm besitzt. Es ist bevorzugt, wenn das partikelförmige Siliziumdioxid maximal einen mittleren Teilchendurchmesser von 500 µm besitzt.

Ferner ist es erfindungsgemäß vorteilhaft, wenn das partikelförmige Siliziumdioxid Poren, insbesondere Meso- und/oder Makroporen, aufweist. Das Volumen dieser Poren nimmt bevorzugt 10 bis 80 % des Volumens eines Siliziumdioxid-Partikels ein. Besonders bevorzugt ist es, wenn die Summe des Volumens derjenigen Poren mit einem Durchmesser kleiner als 5 nm nicht mehr als 5 % des gesamten Porenvolumens ausmacht.

Es ist bevorzugt, wenn das partikelförmige Siliziumdioxid ein Porenvolumen von 0.5 bis 3.0 mUg besitzt.

Beispiele des erfindungsgemäß verwendbaren Siliziumdioxids werden in den beiden Druckschriften WO-03/037287 und EP-A1-725 037 beschrieben, auf die hier ausdrücklich und vollinhaltlich Bezug genommen wird.

Es ist bevorzugt, die Menge des verwendeten partikelförmigen Siliziumdioxids an die Menge des Peroxid-haltigen Pulvers anzupassen. Dabei ist es vorteilhaft, wenn das partikelförmige Siliziumdioxid in einer solchen Menge verwendet wird, dass in dem resultierenden entstaubten Blondierpulver der Anteil von feinerem Korn mit einem mittleren Teilchendurchmesser von kleiner 20 µm, insbesondere kleiner 40 µm, nicht mehr als 5 Gew.-%, jeweils bezogen auf das Gewicht des Blondierpulvers, beträgt.

Bevorzugt werden 1 bis 4 Gew.-%, bezogen auf das Gewicht des Blondierpulvers, des partikelförmigen Siliziumdioxids in den Blondierpulvern verwendet.

Erfindungsgemäß einsetzbares partikelförmiges Siliziumdioxid wird als Versuchsprodukt beispielsweise von der Degussa AG unter den Handelsnamen VP Aeroperl^{®} 300, VP Aeroperl^{®} 300/30, VP Aeroperl^{®} 806/30 oder VP Aeroperl^{®} 50/25 als granuliertes Silika vertrieben. Die erfindungsgemäße Anforderung der bevorzugten Korngrößen kann durch Sieben des handelsüblichen, partikelförmigen Siliziumdioxids eingestellt werden.

Das Pulver enthält als Peroxid-Komponente zwingend mindestens eine Peroxoverbindung. Dies sind in der Regel solche Peroxoverbindungen, die keine Anlagerungsprodukte von Wasserstoffperoxid an andere Komponenten darstellen. Die Auswahl der in den erfindungsgemäßen Mitteln potentiell enthaltenen Peroxoverbindungen unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat und Peroxide wie Magnesium- und Bariumperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

In einer weiteren Ausführungsform enthalten die erfindungsgemäßen Peroxid-haltigen Pulver mindestens ein Alkalisierungsmittel. Erfindungsgemäß können die dem Fachmann für Bleichmittel bekannten, üblichen Alkalisierungsmittel wie Ammonium-, Alkalimetall- und Erdalkalimetallhydroxide, -carbonate, -hydrogencarbonate, - hydroxycarbonate und -carbamide, sowie Alkaliphosphate verwendet werden.

In einer bevorzugten Ausführungsform enthalten die erfindungsgemäßen Peroxid-haltigen Pulver mindestens ein Alkalisierungsmittel, ausgewählt aus Ammonium-, Alkalimetall- und Erdalkalimetall-carbonaten, -hydrogencarbonaten, -metasilikaten und - carbamiden.

Ein zweiter Gegenstand der Erfindung ist die Verwendung von partikelförmigem Siliziumdioxid mit einem mittleren Teilchendurchmesser von mindestens 20 µm und einer BET-Oberfläche von 40 bis 400 m²/g (bestimmt nach DIN 66131 mit Stickstoff) zum Entstauben von Peroxid-haltigen Pulvern.

Die bevorzugten Parameter und Definitionen des ersten Erfindungsgegenstandes gelten auch für diesen Erfindungsgegenstand.

Ein dritter Gegenstand der vorliegenden Erfindung sind Blondierpulver, enthaltend
(a) mindestens eine Peroxoverbindung und
(b) partikelförmiges Siliziumdioxid mit einem mittleren Teilchendurchmesser von mindestens 20 µm und einer BET-Oberfläche von 40 bis 400 m²/g (bestimmt nach DIN 66131 mit Stickstoff).

Die bevorzugten Parameter und Definitionen des ersten Erfindungsgegenstandes gelten auch für diesen Erfindungsgegenstand.

Der vierte Erfindungsgegenstand ist ein Verfahren zur Herstellung eines Bleichmittels für keratinhaltige Fasern, insbesondere menschliche Haare, in dem eine Oxidationsmittel-haltige, wässrige Zusammensetzung mit einem Blondierpulver, enthaltend
(a) mindestens eine Peroxoverbindung und
(b) partikelförmiges Siliziumdioxid mit einem mittleren Teilchendurchmesser von mindestens 20 µm und einer BET-Oberfläche von 40 bis 400 m²/g (bestimmt nach DIN 66131 mit Stickstoff),
gemischt wird.

Die bevorzugten Parameter und Definitionen des ersten, zweiten und dritten Erfindungsgegenstandes gelten auch für diesen Erfindungsgegenstand.

Unter keratinhaltigen Fasern werden erfindungsgemäß Pelze, Wolle, Federn und insbesondere menschliche Haare verstanden.

Die in dem erfindungsgemäßen Verfahren des dritten Erfindungsgegenstandes verwendeten Oxidationsmittel-haltigen, wässrigen Zusammensetzungen enthalten ein solches Oxidationsmittel, welches den natürlichen Farbstoff der keratinhaltigen Faser zu oxidieren vermag. Als erfindungsgemäßes Oxidationsmittel wird bevorzugt Wasserstoffperoxid verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon·n H₂O₂ (n ist eine positive ganze Zahl größer 0), Harnstoffperoxid und Melaminperoxid, dem erfindungsgemäßen Bleichmittel zugesetzt.

Die Konzentration des Wasserstoffperoxids in der wässrigen Zusammensetzung wird einerseits von den gesetzlichen Vorgaben und andererseits von dem gewünschten Effekt bestimmt; in der Regel werden 6- bis 12-prozentige Lösungen in Wasser verwendet.

Eine wässrige Zusammensetzung enthält mindestens 40 Gew.-% Wasser.

Die Oxidationsmittel-haltige, wässrige Zusammensetzung und das Bleichpulver werden üblicherweise in Mengenverhältnissen im Bereich von 1.5:1 bis 1:2 gemischt, wobei ein Überschuß an der Oxidationsmittel-haltigen, wässrigen Zusammensetzung insbesondere dann gewählt wird, wenn keine zu ausgeprägte Blondierwirkung erwünscht ist.

Die Mischung der Komponenten läuft ohne nennenswerte Staubbildung ab und bietet so eine Verbesserung des üblichen Herstellungsverfahrens bekannter Aufhellmittel für keratinhaltige Fasern.

Ein fünfter Erfindungsgegenstand ist ein Mittel zur Aufhellung keratinhaltiger Fasern, insbesondere menschlicher Haare, enthaltend
(a) mindestens ein Oxidationsmittel,
(b) mindestens eine Peroxoverbindung und
(c) partikelförmiges Siliziumdioxid mit einem mittleren Teilchendurchmesser von mindestens 20 µm und einer BET-Oberfläche von 40 bis 400 m²/g (bestimmt nach DIN 66131 mit Stickstoff).

Die bevorzugten Parameter und Definitionen des ersten, zweiten, dritten und vierten Erfindungsgegenstandes gelten auch für diesen Erfindungsgegenstand.

Die Peroxoverbindungen sind in den erfindungsgemäßen Bleichmitteln bevorzugt in Mengen von 1-40 Gew.-%, insbesondere in Mengen von 2-30 Gew.-%, enthalten.

Die erfindungsgemäßen Bleichmittel enthalten die im ersten Erfindungsgegenstand definierten Alkalisierungsmittel bevorzugt in Mengen von 0.2 bis 25 Gew.-%, insbesondere 0.5 bis 10 Gew.-%.

Der pH-Wert der erfindungsgemäßen Mittel liegt bevorzugt in einem pH-Bereich von pH 2.5 bis 12.0, besonders bevorzugt von pH 4.5 bis 10.0.

Als zusätzliche Bleichverstärker können die erfindungsgemäßen Mittel bevorzugt mindestens eine organische Verbindung mit einer Carboxylgruppe, bevorzugt ausgewählt aus Essigsäure, Milchsäure, Weinsäure, Zitronensäure, Salicylsäure und ortho-Phthalsäure, enthalten.

In einer weiteren Ausführungsform enthalten die erfindungsgemäßen Bleichmittel zusätzlich weitere strukturverbessernde Wirkstoffe. Solche die Haarstruktur verbessernden Wirkstoffe stellen Vitamine und deren Derivate beziehungsweise Vorstufen dar. Erfindungsgemäß besonders bevorzugt sind Panthenol und seine physiologisch verträglichen Derivate. Solche Derivate sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 A1 offenbarten kationischen Panthenolderivate. Ein erfindungsgemäß bevorzugtes Panthenolderivat ist ferner dessen Vorstufe Pantolacton. Panthenol ist innerhalb dieser Gruppe bevorzugt. Ein weiteres Beispiel für ein strukturverbesserndes Vitamin ist Pyridoxin (Vitamin B6).

Weiterhin ist auch Polyvinylpyrrolidon (PVP) für seine faserstrukturverbessernden Eigenschaften bekannt und erfindungsgemäß bevorzugt.

Weitere, erfindungsgemäß besonders wirksame, strukturverbessernde Verbindungen stellen die Aldehyde dar. Besonders bevorzugte Beispiel sind Formaldehyd und Formaldehyd-abspaltende Verbindungen, wie beispielsweise Methoxymethylester, Dimethylol (thio) harnstoffderivate, Oxazolidinderivate, N-Hydroxymethylmaleinimid, Hexamethylentetramin und seine Derivate, Hydantoinderivate, Pyridinium-substituierte Dimethylether, Imidazolidinylharnstoff-Derivate, Isothiazolinone, 2-Brom-2-nitropropandiol und 5-Brom-5-nitro-1,3-dioxan. Weitere besonders bevorzugte Aldehyde sind Acetaldehyd, Glyoxal, Glycerinaldehyd und Glutardialdehyd.

Eine weitere geeignete Gruppe von strukturverbessernden Wirkstoffen sind Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, grünem Tee, Ginseng und Ingwerwurzel bevorzugt.

Besonders bevorzugt sind die Extrakte aus Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, grünem Tee, Ginseng und Ingwerwurzel.

Ganz besonders für die erfindungsgemäßen Mittel geeignet sind die Extrakte aus Mandel, Aloe Vera, Kokosnuß, Mango, Aprikose, Limone, Weizen, Kiwi, Melone und grünem Tee.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Ebenfalls erfindungsgemäß als strukturverbessernde Wirkstoffe bevorzugt sind Honigextrakte. Diese Extrakte werden in analoger Weise zu den Pflanzenextrakten gewonnen und enthalten üblicherweise 1 - 10 Gew.-%, insbesondere 3 - 5 Gew.-%, Aktivsubstanz. Wasser/Propylenglykol-Mischungen können auch hier bevorzugte Extraktionsmittel sein.

Weitere strukturverbessernde Wirkstoffe sind Proteinhydrolysate, insbesondere Elastin-, Kollagen-, Keratin-, Milcheiweiß-, Sojaprotein-, Mandelprotein- und Weizenproteinhydrolysate, deren Kondensationsprodukte mit Fettsäuren sowie quaternisierte Proteinhydrolysate. Besonders bevorzugt sind stark abgebaute Keratinhydrolysate mit Molmassen im Bereich von 400 bis 800. Ferner sind quaternierte Proteinhydrolysate, wie sie beispielsweise unter den Handelsbezeichnungen Gluadin^{®} WQ (INCI-Bezeichnung: Laurdimonium Hydroxypropyl Hydrolyzed Wheat Protein) und Crotein^{®} Q (INCI-Bezeichnung: Hydroxypropyltrimonium Hydrolyzed Collagen) vertrieben werden, erfindungsgemäß besonders bevorzugt.

Neben den quaternierten Proteinhydrolysaten stellen auch quaternäre Polymere erfindungsgemäß bevorzugte strukturverbessernde Verbindungen dar. Besonders bevorzugt sind die Polymere, die unter den Handelsbezeichnungen Mirapol^{®} A15 (INCI-Bezeichnung: Polyquaternium-2), Onamer^{®} M (INCI-Bezeichnung: Polyquaternium-1) und Merquat^{®} 100 (INCI-Bezeichnung: Polyquaternium-6), Polymer JR 400 (INCI-Bezeichnung: Polyquaternium-10) vertrieben werden.

Ebenfalls faserstrukturverbessernde Wirkstoffe sind Mono-, Di- und Oligosaccharide wie beispielsweise Glucose, Galactose, Fructose, Fruchtzucker, Saccharose und Lactose. Weiterhin können auch Derivate dieser Pentosen, Hexosen und Heptosen, wie die entsprechenden On- und Uronsäuren (Zuckersäuren), Zuckeralkohole, Zuckeramine, wie beispielsweise N-Glucosamin, und Glykoside, sowie mit C₄-C₃₀-Fettalkoholen veretherte Pentosen, Hexosen und Heptosen, erfindungsgemäß eingesetzt werden. Die Zuckersäuren können erfindungsgemäß in freier Form, in Form ihrer Salze, bevorzugt sind Calcium-, Magnesium- und Zink-Salze, und in Form ihrer Ester oder Lactone eingesetzt werden. Bevorzugte Zuckersäuren sind die Gluconsäure, Gluconsäure-γlacton, Lactobionsäure, die Glucuronsäure und ihre Mono- beziehungsweise Dilactone, die Pangaminsäure, die Zuckersäure, die Mannozuckersäure und ihre Monobeziehungsweise Dilactone sowie die Schleimsäure und ihre Mono- beziehungsweise Dilactone. Bevorzugte Zuckeralkohole sind Sorbit, Mannit und Dulcit. Bevorzugte Glykoside sind die Methylglucoside. Glucose, N-Glucosamin und Gluconsäure sind aus dieser Gruppe besonders bevorzugt.

Auch gewisse Aminosäuren sind in Rahmen der vorliegenden Erfindung als haarstrukturverbessernde Wirkstoffe einsetzbar. Beispiele sind die in der DE-195 22 569, auf die hier ausdrücklich Bezug genommen wird, beschriebenen Aminosäuren Serin, Threonin und Tyrosin. Ferner sind auch Derivate des Serins, wie beispielsweise das Serinphosphat, erfindungsgemäß bevorzugt. Eine weitere strukturverbessernde Aminosäure stellt Lysin dar. Serin ist ein besonders bevorzugter faserstrukturverbessernder Wirkstoff.

Ebenfalls zur Strukturverbesserung können bestimmte Säuren, insbesondere α-Hydroxycarbonsäuren, und ihre Salze eingesetzt werden. Erfindungsgemäß bevorzugte strukturverbessernde Säuren sind Milchsäure, Äpfelsäure, Weinsäure, Glycerinsäure und Maleinsäure. Milchsäure und Glycerinsäure sind besonders bevorzugt. Weiterhin verbessern spezielle Phosphonsäuren und ihre Salze die Struktur keratinhaltiger Fasern.

Erfindungsgemäß bevorzugte Phosphonsäuren sind die n-Octylphosphonsäure und die n-Decylphosphonsäure.

Weiterhin sind lipidlösliche Esteralkohole oder Esterpolyole für ihre strukturverbessernde Wirkung bekannt. Als lipidlöslich sind sie dann anzusehen, wenn sich 5 Gew.-% dieser Produkte in Cetylalkohol bei 80° C klar auflösen.

Die erfindungsgemäß geeigneten Esteralkohole oder Esterpolyole sind erhältlich durch Umsetzung eines Epoxyfettsäureesters mit Wasser oder ein- oder mehrwertigen Alkoholen mit 1 - 10 C-Atomen unter Öffnung des Epoxidrings und Ausbildung einer vizinalen Dihydroxyethyl- oder Hydroxy-alkoxy-ethylgruppe. Der Epoxyfettsäureester kann dabei auch ein Epoxidationsprodukt aus einem technischen Fettsäureester mit Anteilen gesättigter Fettsäuren sein. Der Epoxidsauerstoffgehalt sollte aber wenigstens 3 Gew.-%, bevorzugt 5 - 10 Gew.-%, betragen.

Die Epoxyfettsäureester sind dabei entweder epoxidierte Fettsäureester einwertiger Alkohole, also z.B. epoxidierter Ölsäuremethylester, Linolsäuremethylester, Ricinolsäuremethylester oder epoxidierte Fettsäureester mehrwertiger Alkohole, z.B. Glycerinmonooleat oder Propylenglycol-monooleat oder epoxidierte Fettsäuretriglyceride, z.B. Ölsäuretriglycerid oder ungesättigte Öle wie z.B. Olivenöl, Sojaöl, Sonnenblumenöl, Leinöl, Rüböl.

Technisch besonders interessant sind vor allem ungesättigte Fettsäuremethylester-Epoxide aus ungesättigten Pflanzenfettsäuren. So ist als Esterpolyol das Umsetzungsprodukt eines Pflanzenölfettsäuremethylester-Epoxidats mit einem Polyol mit 2 - 6 C-Atomen und 2 - 6 Hydroxylgruppen besonders bevorzugt. Als Polyole können dabei z.B. Ethylenglycol, 1,2-Propylenglycol, 1,3-Propylenglycol, Butandiol, Pentandiol, Hexandiol, Glycerin, Trimethylolpropan, Pentaerythrit, Sorbit oder Diglycerin enthalten sein.

Besonders gut eignet sich dabei für die erfindungsgemäßen Bleichmittel als Esterpolyol das Umsetzungsprodukt eines Pflanzenfettsäuremethylester-Epoxidats mit Trimethylpropan und mit einer Hydroxylzahl von 350 - 450. Ein solches Produkt auf Basis von Sojaölfettsäuremethylester-Epoxid und Trimethylolpropan ist unter der Handelsbezeichnung Sovermol^{®}760 erhältlich.

Weiterhin kann als strukturverbessernder Wirkstoff Vitamin B₃ eingesetzt werden. Unter dieser Bezeichnung werden häufig die Verbindungen Nicotinsäure und Nicotinsäureamid (Niacinamid) geführt. Erfindungsgemäß bevorzugt ist das Nicotinsäureamid.

Auch Vitamin H ist als strukturverbessernder Wirkstoff im Sinne der vorliegenden Erfindung einsetzbar. Als Vitamin H wird die Verbindung (3a*S*,4*S*, 6a*R*)-2-Oxohexa-hydrothienol[3,4-*d*]-imidazol-4-valeriansäure bezeichnet, für die sich aber zwischenzeitlich der Trivialname Biotin durchgesetzt hat.

Erfindungsgemäß besonders bevorzugte strukturverbessernde Wirkstoffe sind ausgewählt aus Panthenol, physiologisch verträglichen Panthenol-Derivaten, Mono-, Di-und Oligosacchariden, Serin, Milchsäure, Glycerinsäure, Niacinamid, Vitamin B6, Polyvinylpyrrolidon, Glucose, Gluconsäure, Biotin und den lipidlöslichen Esteralkoholen oder Esterpolyolen.

Die erfindungsgemäßen Mittel enthalten die strukturverbessernden Wirkstoffe bevorzugt in Mengen von 0,1 bis 5 Gew.-%, besonders bevorzugt in Mengen von 0,2 bis 2 Gew.-%.

In einer bevorzugten Ausführungsform der vorliegenden Erfindung enthalten die Mittel weiterhin eine Magnesiumverbindung. Die erfindungsgemäßen Mittel können durch Zugabe der Mg²⁺-Kationen hinsichtlich ihre strukturerhaltenden Eigenschaften weiter optimiert werden. Bevorzugte Magnesiumverbindungen sind anorganische und organische Mg²⁺-Salze, wie beispielsweise die Halogenide, die Carbonate und Hydrogencarbonate, das Acetat und das Citrat.

Die erfindungsgemäßen Mittel können weiterhin alle für solche Zubereitungen bekannten Wirk-, Zusatz- und Hilfsstoffe enthalten. In vielen Fällen enthalten die Bleichmittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol-oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾ oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Bleichmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.
Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkyl-ammoniummethosulfate sowie die unter dem Warenzeichen Dehyquart^{®} vertriebenen Produkte wie Dehyquart^{®} AU-46.

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfindungsgemäßen Bleichmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.

### Bevorzugte kationische Polymere sind beispielsweise

- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden.
- quaternierter Polyvinylalkohol sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22. Als konditionierende Wirkstoffe weiterhin geeignet sind Silikonöle, insbesondere Dialkyl-und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abi^{®}-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl, sowie Tocopherolacetat.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyltrimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle,
- Cyclodextrine,
- Lösungsmittel und -vermittler wie Ethanol, Isopropanol, Ethylenglykol, Propylenglykol, Glycerin, Dimethylisosorbid und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die folgenden Beispiele sollen den Gegenstand der vorliegenden Erfindung erläutern ohne ihn jedoch zu beschränken.

### Beispiele

Es wurden folgende Blondierpulver durch vermischen der Komponenten gemäß Tabelle 1 hergestellt.

**Tabelle 1:**

| | erfindungsgemäß | | | nicht erfindungsgemäß | | |
|---|---|---|---|---|---|---|
| Rohstoff | 1 | 2 | 3 | 4 | 5 | 6 |
| Ammoniumperoxidisulfat | 3.0 g | 32.5 g | - | 3.0 g | 32.5 g | - |
| Kaliumperoxidisulfat | 30.0 g | 15.0 g | 27.0 g | 30.0 g | 15.0 g | 27.0 g |
| Natriumperoxidisulfat | 30.0 g | 15.0 g | 30.0 g | 30.0 g | 15.0 g | 30.0 g |
| Natriummetasilikat | 20.0 g | 20.0 g | 20.0 g | 20.0 g | 20.0 g | 20.0 g |
| Ammoniumchlorid | - | - | 5.0 g | - | - | 5.0 g |
| Natriumstearat | 11.0 g | 11.0 g | 11.0 g | 11.0 g | 11.0 g | 11.0 g |
| VP Aeroperl^{®} 300/30 ¹ | 2.5 g | 3.0 g | 3.5 g | - | - | - |
| Aerosil^{®} 200 ² | - | - | - | 2.5 g | 3.0 g | 3.5 g |
| Dinatrium-EDTA | 1.0 g | 1.0 g | 1.0 g | 1.0 g | 1.0 g | 1.0 g |
| Trinatriumphosphat | 2.0 g | 2.0 g | 2.0 g | 2.0 g | 2.0 g | 2.0 g |
| Xanthan Gum | 0.5 g | 0.5 g | 0.5 g | 0.5 g | 0.5 g | 0.5 g |

| | | | | | | |
|---|---|---|---|---|---|---|
| ¹ pyrogenes Siliziumdioxid mit einem mittleren i eilcnenaurcnmesser von 40 µm und einer BET-Oberfläche von ca. 300 m²/g (INCI-Bezeichnung: Silica) (Degussa) ² pyrogenes Siliziumdioxid mit einem mittleren Teilchendurchmesser von 12 nm und einer BET-Oberfläche von 200 m²/g (INCI-Bezeichnung: Silica) (Degussa) | | | | | | |

Zur Beurteilung des Staubverhaltens wurden die Rezepturen in jeweils ein 250 mL Schraubdeckelglas aus ungefärbtem, klarem Glas gefüllt, so daß das Schraubdeckelglas bis zur Hälfte gefüllt war. Danach wurde jedes Schraubdeckelglas mit dem Schraubverschluß verschlossen und kurz geschüttelt. Das Absetzverhalten der jeweiligen Rezepturen nach dem Schütteln wurde beurteilt. Die erfindungsgemäßen Rezepturen 1 bis 3 setzten sich nach wenigen Sekunden nahezu vollständig am Boden ab. Es war kein Staub in der oberen Hälfte des Schraubdeckelglases zu sehen.

Die Vergleichsrezepturen 4 bis 6 besaßen nach dem Schütteln noch nach einer Minute einen deutlich sichtbaren Staubanteil im überstehendem Luftraum der oberen Hälfte des Schraubdeckelglases.

## Patentansprüche

1. Verfahren zur Entstaubung von Blondierpulvern, worin ein Peroxid-haltiges Pulver mit partikelförmigen Siliziumdioxid vermengt wird, **dadurch gekennzeichnet, dass** das partikelförmige Siliziumdioxid Poren aufweist und einen mittleren Teilchendurchmesser von mindestens 20 µm und eine BET-Oberfläche von 40 bis 400 m²/g (bestimmt nach DIN 66131 mit Stickstoff) besitzt.

2. Verfahren nach Anspruch 1, **dadurch gekennzeichnet, dass** das partikelförmige Siliziumdioxid Meso- und/oder Makroporen aufweist.

3. Verfahren nach einem der Ansprüche 1 oder 2, **dadurch gekennzeichnet, dass** das partikelförmige Siliziumdioxid ein Porenvolumen von 0.5 bis 3.0 mL/g besitzt.

4. Verfahren nach einem der Ansprüche 1 bis 3, **dadurch gekennzeichnet, dass** die Summe des Volumens derjenigen Poren mit einem Durchmesser kleiner als 5 nm nicht mehr als 5 % des gesamten Porenvolumens ausmacht.

5. Blondierpulver, enthaltend
(a) mindestens eine Peroxoverbindung und
(b) partikelförmiges Siliziumdioxid, welches Poren aufweist, mit einem mittleren Teilchendurchmesser von mindestens 20 µm und einer BET-Oberfläche von 40 bis 400 m²/g (bestimmt nach DIN 66131 mit Stickstoff).

6. Verfahren zur Herstellung eines Bleichmittels für keratinhaltige Fasern, insbesondere menschliche Haare, in welchem eine Oxidationsmittel-haltige, wässrige Zusammensetzung mit einem Blondierpulver, enthaltend
(a) mindestens eine Peroxoverbindung und
(b) partikelförmiges Siliziumdioxid, welches Poren aufweist, mit einem mittleren Teilchendurchmesser von mindestens 20 µm und einer BET-Oberfläche von 40 bis 400 m²/g (bestimmt nach DIN 66131 mit Stickstoff),
gemischt wird.

7. Mittel, enthaltend
(a) mindestens ein Oxidationsmittel,
(b) mindestens eine Peroxoverbindung und
(c) partikelförmiges Siliziumdioxid, welches Poren aufweist, mit einem mittleren Teilchendurchmesser von mindestens 20 µm und einer BET-Oberfläche von 40 bis 400 m²/g (bestimmt nach DIN 66131 mit Stickstoff).

8. Mittel nach Anspruch 7, **dadurch gekennzeichnet, dass** das Oxidationsmittel Wasserstoffperoxid ist.

9. Mittel nach einem der Ansprüche 7 oder 8, **dadurch gekennzeichnet, dass** es zusätzlich mindestens ein Tensid, ausgewählt aus der Gruppe der anionischen, zwitterionischen, ampholytischen, nichtionischen oder kationischen Tenside, enthält.

## Claims

1. A method for dedusting blonding powders, wherein a peroxide-containing powder is mixed with particulate silicon dioxide, **characterized in that** the particular silicon dioxide has pores and an average particle diameter of at least 20 µm and a BET surface area from 40 to 400 m²/g (determined according to DIN 66131 with nitrogen).

2. The method according to claim 1, **characterized in that** the particular silicon dioxide has meso- and/or macro-pores.

3. The method according to any of claims 1 or 2, **characterized in that** the particular silicon dioxide has a pore volume from 0.5 to 3.0 mUg.

4. The method according to any of claims 1 to 3, **characterized in that** the sum of the volume of those pores with a diameter of less than 5 nm does not constitute more than 5% of the whole pore volume.

5. A blonding powder, containing
(a) at least one peroxo compound and
(b) particulate silicon dioxide, which has pores, with an average particle diameter of at least 20 µm and a BET surface area from 40 to 400 m²/g (determined according to DIN 66131 with nitrogen).

6. A method for preparing a bleaching agent for keratinous fibers, in particular human hair, in which an aqueous oxidant-containing composition is mixed with a blonding powder, containing
(a) at least one peroxo compound and
(b) particulate silicon dioxide, which has pores, with an average particle diameter of at least 20 µm and a BET surface area from 40 to 400 m²/g (determined according to DIN 66131 with nitrogen).

7. An agent, containing
(a) at least one oxidant,
(b) at least one peroxo compound and
(c) particular silicon dioxide, which has pores, with an average particle diameter of at least 20 µm and a BET surface area from 40 to 400 m²/g (determined according to DIN 66131 with nitrogen).

8. The agent according to claim 7, **characterized in that** the oxidant is hydrogen peroxide.

9. The agent according to any of claims 7 or 8, **characterized in that** it further contains at least one surfactant, selected from the group of anionic, zwitterionic, ampholytic, non-ionic or cationic surfactants.

## Revendications

1. Procédé pour dépoussiérer des poudres de décoloration, dans lequel on mélange une poudre contenant un peroxyde avec du dioxyde de silicium sous forme de particules, **caractérisé en ce que** le dioxyde de silicium sous forme de particules présente des pores et un diamètre moyen de particules d'au moins 20 µm et une surface BET de 40 à 400 m²/g (déterminée selon la norme DIN 66131 avec de l'azote).

2. Procédé selon la revendication 1, **caractérisé en ce que** le dioxyde de silicium sous forme de particules présente des mésopores et/ou des macropores.

3. Procédé selon l'une quelconque des revendications 1 ou 2, **caractérisé en ce que** le dioxyde de silicium sous forme de particules présente un volume de pores de 0,5 à 3,0 ml/g.

4. Procédé selon l'une quelconque des revendications 1 à 3, **caractérisé en ce que** la somme du volume des pores présentant un diamètre inférieur à 5 nm n'est pas supérieure à 5% du volume total des pores.

5. Poudre de décoloration, contenant
(a) au moins un composé peroxo et
(b) du dioxyde de silicium sous forme de particules qui présente des pores, présentant un diamètre moyen de particules d'au moins 20 µm et une surface BET de 40 à 400 m²/g (déterminée selon la norme DIN 66131 avec de l'azote).

6. Procédé pour la préparation d'un agent de décoloration pour des fibres kératiniques, en particulier des cheveux humains, dans lequel on mélange une composition aqueuse contenant un oxydant, avec une poudre de décoloration, contenant
(a) au moins un composé peroxo et
(b) du dioxyde de silicium sous forme de particules qui présente des pores, présentant un diamètre moyen de particules d'au moins 20 µm et une surface BET de 40 à 400 m²/g (déterminée selon la norme DIN 66131 avec de l'azote).

7. Agent, contenant
(a) au moins un oxydant,
(b) au moins un composé peroxo et
(c) du dioxyde de silicium sous forme de particules qui présente des pores, présentant un diamètre moyen de particules d'au moins 20 µm et une surface BET de 40 à 400 m²/g (déterminée selon la norme DIN 66131 avec de l'azote).

8. Agent selon la revendication 7, **caractérisé en ce que** l'oxydant est du peroxyde d'hydrogène.

9. Agent selon l'une quelconque des revendications 7 ou 8, **caractérisé en ce qu'**il contient en outre au moins un agent tensioactif, choisi dans le groupe des agents tensioactifs anioniques, zwittérioniques, ampholytiques, non ioniques ou cationiques.
